Europäisches Patentamt

European Patent Office

Office européen des brevets

(11) Publication number: **0 405 763 B1**

## EUROPEAN PATENT SPECIFICATION

(45) Date of publication of patent specification: **29.03.95**   (51) Int. Cl.6: **C07K 7/08**, C12N 15/16, C12P 21/02, A61K 38/04

(21) Application number: **90306033.3**

(22) Date of filing: **01.06.90**

(54) **GRF peptides.**

(30) Priority: **03.06.89 GB 8912837**

(43) Date of publication of application:
**02.01.91 Bulletin 91/01**

(45) Publication of the grant of the patent:
**29.03.95 Bulletin 95/13**

(84) Designated Contracting States:
**AT BE CH DE DK ES FR GB GR IT LI LU NL SE**

(56) References cited:
**EP-A- 0 171 315**

**PROCEEDINGS OF THE NATIONAL ACADEMY OF SCIENCES, USA, vol. 82, January 1985, pages 63-67, Washington, DC, US; K.E. MAYO et al.: * Page 65, figure 3 ***

(73) Proprietor: **MALLINCKRODT VETERINARY LIMITED**
**Breakspear Road South**
**Harefield**
**Uxbridge**
**Middlesex UB9 6LS (GB)**

(72) Inventor: **Stephen, James, Coopers Animal Health Limited**
**Berkhamsted Hill**
**Berkhamsted,**
**Hertfordshire (GB)**

(74) Representative: **Matthews, Derek Peter et al**
**Frank B. Dehn & Co.**
**Imperial House**
**15-19 Kingsway**
**London WC2B 6UZ (GB)**

Rank Xerox (UK) Business Services
(3.10/3.09/3.3.3)

EP 0 405 763 B1

**Description**

The present invention relates to biologically active peptides.

Many polypeptide hormones such as growth hormones are important medically or in animal husbandry. Growth hormones are found in all vertebrates and are known, for example, to enhance growth (somatogenesis) and to enhance milk production (lactogenesis). The control and release of growth hormone (GH) is dependent on other hormones or "factors" which can result in either increased or decreased amounts of growth hormone in the vertebrate. Growth hormone release is in response to the effect of a growth hormone releasing peptide known as growth hormone releasing factor (GRF), growth hormone releasing hormone (GHRH) or somatocrinin.

Two original reports from the Salk Institute described a growth hormone releasing peptide of up to 44 amino-acids from a human pancreatic (hp) tumour (Rivier et al 1982; Guillemin et al 1982). It specifically releases growth hormones at physiological doses and has been shown to be identical to the GH releasing hormone normally secreted from the hypothalamus and exerting its activity at the pituitary somatotrophs. It is believed that the balance of somatostatin (somatotrophin inhibiting factor) and GRF is the primary influence upon the pituitary giving the characteristic GH release profile of mammals.

The amino acid sequence of GRF from a number of species has been described (Felix et al 1985). These are 44 amino acid peptides, the most highly conserved region is from residues 1-27 with most variation residing on the residues 31-44. There is considerable cross-species activity of the full sequence, for instance hpGRF-44 elicits good response in goats (Hart et al 1984). Furthermore the activity of the molecule appears to be most importantly located in the 1-27 region and a number of analogues of this region have shown improved activity in model species (Lance et al 1984). As well as use in humans, administration of hPGRF to cattle can improve milk yield (Enright et al 1986) and increase nitrogen retention (Moseley et al 1987) via its GH releasing activity.

Species Specific Sequences (from Felix et al 1985)

1 -27 common sequence : YADAIFTNSYRKVLGQLSARKLLQDIM
28-44 human: GRF : SRQQGESNQERGARARL-NH$_2$
porcine: GRF : SRQQGERNQEQGARVRL-NH$_2$
bovine: GRF : NRQQGERNQEQGAKVRL-NH$_2$
caprine: GRF : NRQQGERNQEQGAKVRL-NH$_2$
ovine: GRF : NRQQGERNQEQGAKVRL-NH$_2$

(Letters are standard single letter notations for amino acids - see Appendix I).

It has been reported that the activity of hormones can be affected by antibodies raised against them. Thus, in EP A-137-234 antibodies to growth hormone isolated and administered to animals resulted in increased activity of the hormone. These antibodies can also be raised in situ by vaccinating the host animal with suitably presented fragments of the hormone in question as shown in EP A-284-406 and EP A-303-488.

However, Armstrong et al (1989) have reported that immunisation against all of the GRF molecule, will inhibit the activity of this hormone. Furthermore, Reynolds et al (1989) have shown that immunisation against certain fragments of corticotrophin releasing factor (CRF) abolished its activity as shown by reduced circulating glucocorticoid concentration.

In contrast, it has now been discovered that the activity of GRF can be potentiated in a vertebrate by administering to that vertebrate a particular fragment of GRF that produces antibodies to the intact GRF. It is believed that this potentiation of GRF will stimulate growth, carcass-composition, milk yield and other biological effects of GH in that vertebrate. It is to be understood that as used herein, the term 'fragment' excludes reference to the whole of the GRF molecule.

Accordingly in a first aspect, the present invention provides a pharmaceutical antigenic formulation comprising a peptide fragment of vertebrate growth hormone releasing factor (GRF) from the region spanning positions 28-44 thereof, or a salt, ester, amide or protected form of said peptide and means to provide adjuvant and carrier functions, wherein said peptide is capable of raising antibodies in a vertebrate to increase or enhance the activity of GRF in said vertebrate. The peptide may be derived from native protein, synthesised chemically or prepared as a recombinant gene product of an appropriate expression system using methods known in the art.

The term "antigenically equivalent" means a peptide which will be specifically recognised by certain antibodies which, when raised to peptides according to the invention, or parts thereof will act to potentiate the biological activity of a growth hormone releasing factor in that or a similar vertebrate. The term "immunogenically equivalent" means that the peptide can be used, in a suitable formulation, to raise antibodies in a vertebrate, the antibodies acting to potentiate the action of the releasing factor in that vertebrate.

Thus antigenically or immunogenically equivalent peptides which are slightly shorter than the said region may be used. Variations from the sequence of the animal's own GRF may cause a greater immune response whilst still yielding antibodies able to recognise the animal's own GRF or targetted GRF (for example one exogenously introduced via an implant). Accordingly, it can be advantageous to administer a small peptide of the invention from a species other than the animal to which the peptide is being administered (for example administering ovine peptides to porcines).

In addition, peptides in which one or more of the amino acid residues are chemically modified, before or after the peptide is synthesised, may be used providing that the function of the peptide, namely the production of the specific antibodies in vivo remains substantially unchanged. Such modifications include forming salts with acids or bases especially physiologically acceptable organic or inorganic acids and bases, forming an ester or amide of a terminal carboxyl group and attaching amino acid protecting groups such as N-t-butoxycarbonyl. Such modifications may protect the peptide from in vivo metabolism. The peptides may be present as single copies or as multiples, for example tandem repeats such as (31-44)+-(31-44) or (31-44)+(44-31). Such tandem or multiple repeats may be sufficiently antigenic themselves to obviate the use of a carrier.

It may be advantageous for the peptide to be formed as a loop, with the N-terminal and C-terminal ends joined together, or to add one or more cysteine residues to an end to increase antigenicity and/or to allow disulphide bonds to be formed. If the peptide is covalently linked to a carrier, preferably a polypeptide, then the arrangement is preferably such that the peptide of the invention forms a loop. Suitably, the peptides are amidated at the C-terminal end and an additional amino acid eg. cysteine added to the N-terminal end to facilitate conjugation or to improve potential immunogenicity.

A second aspect of the invention provides a pharmaceutical antigenic composition comprising one or more of the peptides of the first aspect of the invention with means to provide carrier and adjuvant functions.

According to current immunological theories, a carrier function should be present in any immunogenic formulation in order to stimulate, or enhance stimulation of the immune system. It is thought that carriers embody (or together with the antigen, create) a helper T-cell epitope. The peptides may be associated, for example by cross-linking, with a separate carrier, such as serum albumins, myoglobins, bacterial toxoids and keyhole limpet haemocyanin.

More recently developed carriers which induce T-cell help in the immune response include the hepatitis-B core antigen (also called the nucleocapsid protein), presumed T-cell epitopes such as Thr-Ala-Ser-Gly-Val-Ala-Glu-Thr-Thr-Asn-Cys, betagalactosidase and the 163-171 peptide of interleukin-1. The latter compound may variously be regarded as a carrier or as an adjuvant or as both. Alternatively, several copies of the same or different peptides of the invention may be cross-linked to one another; in this situation there is no separate carrier as such, but a carrier function may be provided by such cross-linking. Suitable cross-linking agents include those listed as such in the Sigma and Pierce catalogues, for example glutaraldehyde, carbodiimide and succinimidyl 4-(N-maleimidomethyl)cyclohexane-1-carboxylate, the latter agent exploiting the -SH group on the C-terminal cysteine residue (if present).

Suitably, cross-linking or conjugation using MBS (m-maleimidobenzoyl-N-hydroxy succinimide) or glutaraldehyde is to a polypepide, for example; the peptides themselves, a T-cell epitope or most suitably Keyhole Limpet Haemocyanin.

If the peptide is prepared by expression of an appropriate nucleotide sequence in a suitable host, then it may be advantageous to express the peptide as a fusion product with a peptide sequence which provides T-cell help and acts as a carrier. Kabigen's "Ecosec" system is an example of such an arrangement. "Ecosec" is a trade name.

Suitable adjuvants are known to those in the vaccine field, for example Freund's complete or incomplete adjuvant, aluminium hydroxide, saponin, DEAE-dextran, muramyl dipeptide, mineral oils, neutral oils (such as miglyol), vegetable oils (such as arachis oil), "Iscoms", liposomes, Novasomes or similar technologies, Pluronic polyols or the Ribi adjuvant system. (See for example GB-A-2189141). "Pluronic" is a Registered Trade Mark.

The peptide of the invention may be linked to other antigens to provide a dual effect. For example, the peptide may be linked to part or all of the somatostatin molecule to create, in addition to anti-GRF antibodies, anti-somatostatin antibodies which would promote growth, or it may be linked to part or all of a sex hormone molecule to provide for simultaneous immunocastration, or to part or all of the luteinising hormone releasing hormone (LHRH), or it may be linked to part or all of the growth hormone molecule to provide for simultaneous potentiation of the action of growth hormone, or any other peptide hormones involved in growth and modulation of carcass composition.

EP 0 405 763 B1

The peptides and adjuvants and/or carriers may be formulated in any suitable way which may be devised by the man skilled in the art using known or yet-to-be-discovered delivery vehicles and criteria. In particular, the formulations may be based on biodegradable polymers such as lactide glycolide copolymers, such as those described in EP-A-58481 (ICI). The peptides of the invention (whether or not linked to other antigens) can be used in combination with other immunisation procedures, (for example against bacterial, viral or parasitic infections) to enhance other peptides.

A further aspect of the invention provides anti-GRF antibodies directed to GRF sequences from the region comprising 28-44, (in particular anti-GRF antibodies directed to the fragment of GRF 31-44 or 35-44) or antigenically or immunologically equivalent peptide or salts thereof. Suitably, these anti-GRF antibodies can be complexed with GRF or fragments of GRF according to the invention.

A further aspect of the invention provides a method of treating a normal or abnormal vertebrate with a small peptide or antigenic composition as described above or anti-GRF antibodies (suitably anti-GRF antibodies pre-complexed with GRF or fragments of GRF), in order to alter the biological characterstics of the vertebrate, in particular those biological effects associated with GH. For example the growth of that vertebrate can be boosted or enhanced beyond normal levels or at an accelerated rate, or abnormally low levels can be brought up to the norm. Similarly milk yield can be boosted or enhanced along with other biological effects associated with GH such as carcass-composition. For example nitrogen retention and the proportion of lean meat to fat may also be enhanced by using such methods. The term "vertebrate" includes humans and non-humans.

The small peptides and antigenic compositions of the invention will usually be administered intravenously, sub-cutaneously or intra-muscularly although intra-nasal, transdermal, oral and rectal routes may be suitable for some formulations of the invention. The antigen may also be administered by expression as a fusion protein by a genetically engineered bacterium, yeast or virus replicating in the host vertebrate. In particular, peptides and antigens of the invention may be presented by utilising a bacterial carrier using methods known in the art, for example using the double aro mutant of Salmonella (see International patent application PCT/GB88/01143 published under International publication no. WO89/05856). The formulation is normally sterile and (for parenteral use) substantially non-pyrogenic and a unit dose typically includes 1 to 1000μg of the small peptide of the invention, typically 10 to 500μg, preferably about 50μg or less. One or more repeat immunisations may be advantageous, as is known in the art of immunology. The formulations may generally be prepared and/or administered by a physician or veterinary surgeon according to his skill and expertise.

A further aspect of the invention provides a process for preparing one of the peptides mentioned above by known methods of peptide synthesis or by appropriate cleavage of the native GRF molecule. Peptide synthesis may be achieved according to the general method of Stewart et al, (1969) or by the methods described by Marglin and Merrifield (1970), and subsequent articles.

For example, in one approach the peptides and salts are formed by the sequential coupling of appropriate amino acids using either classical methods of peptide synthesis or solid phase procedures, or by the initial preparation and subsequent coupling of peptide subunits. Such reactions may be effected by, for example, activating the reacting carboxyl group of the ingoing amino acid and protecting the non-reacting amino and carboxyl groups, and details of suitable activating and protecting (masking) groups and of suitable reaction conditions (both for the coupling reactions and for the removal of protecting groups) giving the minimum of racemisation may be found in the above-referenced literature.

The peptides and salts may thus be prepared by reacting a reagent (I)

$Y^1$ - OH     (I)

wherein $Y^1$ is a partial radical sequence identical with the corresponding N-terminal partial radical sequence of said peptides, with a reagent (II)

H - $Y^2$     (II)

wherein $Y^2$ is identical with the balance of the above-defined product peptide and includes the corresponding C-terminal partial radical sequence thereof, the reagents (I) and (II) being optionally protected and/or activated where and as appropriate; followed as appropriate by deprotection of the product, followed as appropriate by conversion of the product into the free peptide or a salt thereof.

Established methods of peptide synthesis by solid phase and similar techniques are usually not suitable for large scale production (although they may become so in the future) and thus commercial production of the peptides would normally be by cultivation of a suitable organism transformed with a polynucleotide

4

sequence encoding the desired peptide. Therefore, further aspects of the invention include such polynucleotides, transformation and expression vectors carrying such polynucleotides, organisms transformed therewith and processes for cultivating such organisms.

Accordingly, a further aspect of the invention includes a method of preparing a peptide according to the invention by recombinant DNA methodologies known in the art. For example, a method comprising:

(i) transforming an host cell with a vector which incorporates a gene encoding a peptide according to the invention and which is capable, in the host cell, of expressing said peptide;

(ii) culturing the transformed host cell so that the peptide is expressed; and

(iii)recovering the peptide.

Any appropriate host-vector system may be employed. The vector may be a plasmid. In that event, a bacterial or yeast host may be used. Alternatively, the vector may be a viral vector. This may be used to transfect cells of a mammalian cell line in order to cause peptide expression. A further aspect of the invention includes non-human vertebrates whose characteristics have been altered by methods in accordance with the invention.

Examples in accordance with the invention will now be described, with reference to the accompanying drawings in which:

Figure 1    shows the attenuated GRF response in sheep immunised with anti-GRF 1-14 antisera;

Figure 2    shows the GRF-potentiating response in sheep immunised with anti-GRF 31-44 antisera;

Figure 3    shows the GRF-potentiating response in sheep immunised with anti 35-44 antisera;

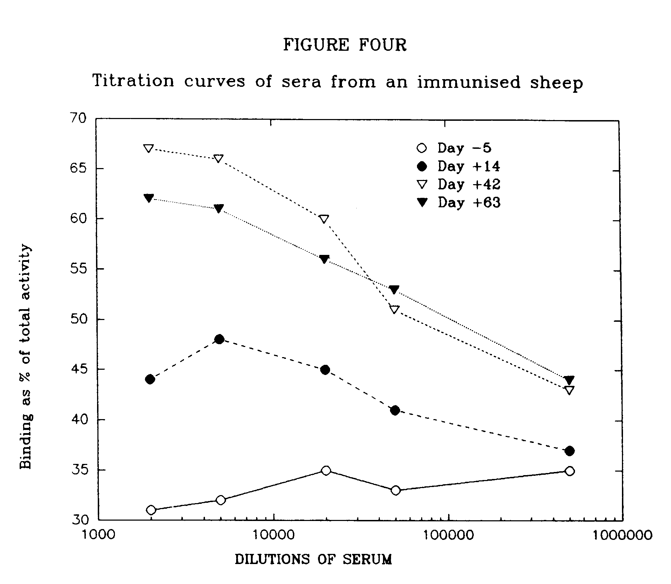

Figure 4    is a titration curve showing the development of an anti-GRF, response in an immunised animal;

Figure 5    summarises the action of antibodies to GRF 1-14, 31-44 or 35-44 upon the biological response of sheep to GRF, over a four hour period and

Figure 6    emphasises the action of antibodies to GRF 1-14, 31-44 or 35-44 during the initial 60 minutes.

GENERAL METHODS

1. Preparation of peptides

Unless otherwise indicated, all peptides were synthesised by the Fmoc-polyamide mode of solid-phase peptide synthesis. Temporary N-α amino group protection is afforded by the 9-fluorenylmethyloxycarbonyl (Fmoc) group. Repetitive cleavage of this highly base-labile protecting group is effected using 20% piperidine in N,N-dimethylformamide.

Side chain functionalities are protected as their butyl ethers (in the case of serine, threonine and tyrosine), butyl esters (in the case of glutamic acid and aspartic acid), butyloxycarbonyl derivative (in the case of lysine and histidine), trityl derivative (in the case of cysteine) and 4-methoxy-2,3,6-trimethylbenzenesulphonyl derivative (in the case of arginine). Where glutamine or asparagine are C-terminal residues, use is made of the 4,4'-dimethoxybenzhydryl group for protection of the side chain amido functionalities.

The solid-phase support is based on a polydimethylacrylamide polymer constituted from the three monomers dimethylacrylamide (backbone-monomer), bis-acryloylethylene diamine (cross linker) and acryloylsarcosine methyl ester (functionalising agent). The peptide-to-resin cleavable linked agent used is the acid-labile 4-hydroxymethyl-phenoxyacetic acid derivative.

All amino acid derivatives are added as their preformed symmetrical anhydride derivatives with the exception of asparagine and glutamine which are added using a reversed N,N-dicyclohexylcarbodiimide/1-hydroxy-benzotriazole mediated coupling procedure.

All coupling and deprotection reactions are monitored using ninhydrin, trinitrobenzene sulphonic acid or isotin test procedures.

Upon completion of synthesis, peptides are cleaved from the resin support with concomitant removal of side-chain protecting groups by treatment with 95% trifluoroacetic acid containing a 5% scavenger mix. Scavengers commonly used are ethanedithiol, phenol, anisole and water, the exact choice depending on the constituent amino acids of the peptide being synthesised. Trifluoroacetic acid is removed by evaporation in vacuo, with subsequent trituration with diethyl ether affording the crude peptide. Any scavengers present are removed by a simple extraction procedure which on lyophilisation of the aqueous phase affords the crude peptide free of scavengers.

Purification may be effected by any one, or a combination of, chromatographic techniques such as size exclusion chromatography, ion-exchange chromatography, affinity chromatography (eg. using an appropriate monoclonal antibody) and reverse-phase high performance liquid chromatography. Analysis of peptides may be carried out using thin layer chromatography, reverse-phase high performance liquid chromatography, amino-acid analysis after acid hydrolysis and by fast atom bombardment (FAB) mass spectrometric analysis.

## 2. Conjugation of peptides

### (i) Conjugation to ovalbumin

3.0mg of peptide was dissolved in 300$\mu$l dimethyl formamide. 150$\mu$l of 10mg/ml ovalbumin in Dulbecco's phosphate buffered saline (PBS) was added and thoroughly mixed. 250$\mu$l of freshly prepared 0.04M glutaraldehyde was added slowly, with stirring, over a period of 10 minutes then left at room temperature for a further 60 minutes with continuous mixing (SPIRAMIX, Denley Instruments). 1.0ml of PBS was added and followed by a further 100$\mu$l 0.04M glutaraldehyde as above. This was left for 60 minutes at room temperature before being dialysed overnight at +4°C against PBS.

### (ii) Conjugation to Keyhole Limpet Haemocyanin (KLH)

5mg of the peptide was conjugated to Keyhole Limpet Haemocyanin using the m-maleimidobenzoyl-N-hydroxy succinimide technique - MBS (Lerner, R.A. et al 1981) supplied by Pierce and Warriner (UK) Ltd., Chester, England, or using the random glutaraldehyde method. The latter was achieved as follows: 5mg of hpGRF peptide was dissolved in 250$\mu$l (PBS (Dulbecco) and 500$\mu$l of KLH 10mg/ml in PBS was mixed. 500$\mu$l of 0.04M glutaraldehyde (Sigma) was added dropwise with agitation. This was rolled at room temperature for 60 minutes. A further 1.5ml of PBS, then 500$\mu$l 0.04M glutaraldehyde were added as above and the mixture rolled for 30 minutes. The reaction mixture was dialysed, using Spectropor dialysis membrane with 1kDa cut off, overnight against 100 volumes of PBS.

## 3. Cross-linking

1.4mg of peptide was dissolved in 140$\mu$l dimethyl formamide and 170$\mu$l of 0.04M glutaraldehyde was added as above. Otherwise as above. If no cross-linking nor conjugation was required the peptide was dissolved in dimethyl formamide, dispersed in PBS but not dialysed.

### Negative Controls

Negative control parallels for the above were produced by using no peptide with ovalbumin (or KLH) or using poly-lysine (molecular weight 1000-2000 Da) and cross-linking as described above.

## 4. Adjuvants and Administration - "Freunds"

After dialysis, the volumes of the above preparations were made up to 4.5ml with PBS and "water-in-oil" emulsions prepared using two volumes of Freunds Complete Adjuvant (Difco or Sigma FCA). This was achieved by sonication in the cold or using a Potter-Elvehjen homogeniser. Emulsions were tested by dispersion (or absence) on a water surface.

The injections were subcutaneously administered at two sites (one on each flank) into Cheviot sheep (9-12 months old, castrate males, 30-35kg). 1ml was administered at each site. A second, similar, immunisation was completed 28-42 days later using freshly prepared peptide conjugated in the same way but emulsified into Freunds Incomplete Adjuvant (FIA, Difco or Sigma). Any subsequent immunisations were similar, but at 28 day intervals. Mice received 0.1ml of the preparations intraperitoneally at similar intervals.

## 5. Adjuvant and Administration - Others

DEAE-dextran (fully hydrated overnight in double-distilled water: Pharmacia), Saponin (Sigma) and aluminium hydroxide ("Al-hydrogel") were used alone and in combinations. After dialysis additions were made of 3.1ml PBS plus 7ml 5% DEAE-dextran (Dd) plus 2.8ml of 5mg/ml saponin; or 5.9ml PBS plus 7ml 5% Dd; or 10.1ml PBS plus 2.8ml of 5mg/ml saponin. Aluminium hydroxide ("AlOH") was used at 1.0mg/ml final concentration where appropriate. No emulsification was required but care was taken to maintain the constituents in homogenous suspension. 1ml was administered into sheep as described above. Immunisations were carried out at the same intervals.

## 6. Blood Samples (Sheep)

10ml blood samples were taken by jugular vene-puncture, from the sheep under test, just prior to any administration and at intervals thereafter. After allowing the clot to form at room temperature (approximately 3-5 hours) the serum was removed after centrifugation for antibody-detecting radio-immunoassay. Larger samples of sera were collected in the same way from approximately 150-200ml of blood, were frozen at-20°C for subsequent fractionation of IgG fraction for use in assays.

## Blood Samples (Mice)

Small blood samples were taken (0.25-0.5ml) from the retro-orbital route or by tail vein section and treated as described for the sheep. Larger samples were not taken.

## 7. Radioimmunoassay

Unless otherwise stated, the detection of antibodies to peptides which would also bind to growth hormone releasing factor was determined by liquid phase direct binding as described previously (Aston et al, 1985; Chard, 1987).

## EXPERIMENTS

## Development of Antisera

## 1A Immunisation of Mice and Sheep against hpGRF 31-44 and detection of hpGRF antibodies

## Method

The following sequence was synthesised by Cambridge Research Biochemicals, Cambridge, England and purified by HPLC to give a peptide in excess of 80% pure.

$$\text{30} \qquad \text{35} \qquad \text{40} \qquad \text{44}$$

$$\text{Cys-Gln-Gly-Glu-Ser-Asn-Gln-Glu-Arg-Gly-Ala-Arg-Ala-Arg-Leu-NH}_2$$

Note that this peptide is amidated at position 44 and a cysteine residue added at position 30 to facilitate conjugation using MBS (see below) or improve potential immunogenicity.

5mg of the peptide was conjugated to Keyhole Limpet Haemocyanin using the m-maleimidobenzoyl-N-hydroxy succinimide technique and the random glutaraldehyde method. The volume of both conjugations was adjusted to 5ml. 10ml of Freund Complete Adjuvant (FCA-Sigma) was added. This was cooled to 0°C and emulsified by sonication using a pre-cooled probe.

The complete preparations were used to immunise two groups of mice and sheep. The latter received 1ml at 2 sub-cutaneous sites on the flank to give a total peptide dose of 667µg/sheep. Five North Cheviot wethers of 18 months old were used per preparation. Six mice (Balb/C) were used per preparation and each received 100µg peptide in FCA, given intraperitoneally. All animals were blood sampled four weeks later and immediately received a similar ("boost") dose to that described previously except that the total peptide loadings were halved and the emulsion was prepared using Freunds Incomplete Adjuvant (FIA-Sigma).

All animals were blood sampled 15 days thereafter and (sheep only) weekly thereafter. All animals received a further boosting dose in FIA 28 days after the first boost. The sera from these blood samples were tested for the presence of anti-GRF antibodies by a competitive radioimmune assay method using 3-[125 I]iodotyrosyl[10] growth hormone releasing factor 1-44 amide (Amersham, Bucks, UK) and anti-mouse or anti-sheep Sac-Cel (Immunodiagnostics Ltd). A rabbit anti-GRF antibody (Metachem Diagnostics Ltd. Northampton, UK) with anti-rabbit Sac-Cel was used as a preliminary positive control in this system. Bulk sheep samples for IgG preparation and subsequent analysis of properties were taken as appropriate.

7

Results

The results are shown in the table indicating the percentage of surviving animals which gave a positive antibody response from at least two of the test bleeds.

| Conjugation Method | Balb/c Mice (survivors) | Cheviot Sheep (survivors) |
|---|---|---|
| MBS | 33% (6/6) | 40% (5/5) |
| Glutaraldehyde | 40% (5/6) | 60% (5/5) |

1B. Immunisation of Sheep against a range of GRF-derived amino-acid sequences

Method

The following sequences were synthesised by Cambridge Research Biochemicals, Cambridge, England and purified by HPLC to give peptides in excess of 80% pure.

$Cys^0 + 1$-14:
(I)      Cys-Tyr-Ala-Asp-Ala-Ile-Phe-Thr-Asn-Ser-Tyr-Arg-Lys-Val-Leu

15-27 + $Cys^{28}$:
(II)      Gly-Gln-Leu-Ser-Ala-Arg-Lys-Leu-Leu-Gln-Asp-Ile-Met-Cys

$Cys^{21} + 22$-35:
(III)      Cys-Leu-Leu-Gln-Asp-Ile-Met-Ser-Arg-Gln-Gln-Gly-Glu-Ser-Asn

$Cys^{30} + 31$-44$NH_2$:
(IV)      Cys-Gln-Gly-Glu-Ser-Asn-Gln-Glu-Arg-Gly-Ala-Arg-Ala-Arg-Leu-$NH_2$

$Cys^{30} + 31$-39:
(V)      Cys-Gln-Gly-Glu-Ser-Asn-Gln-Glu-Arg-Gly

31-39:
(VI)      Gln-Gly-Glu-Ser-Asn-Gln-Glu-Arg-Gly

$Cys^{34} + 35$-44$NH_2$:
(VII)      Cys-Asn-Gln-Glu-Arg-Gly-Ala-Arg-Ala-Arg-Leu-$NH_2$

$Cys^{29} + 30$-43 + $Lys^{44}NH_2$:
(VIII)      Cys-Gln-Gln-Gly-Glu-Arg-Asn-Gln-Glu-Gln-Gly-Ala-Arg-Val-Arg-Lys-$NH_2$.

These peptides were all dissolved in 50mM phosphate buffered saline (pH 7.2, PBS) and conjugated on an equal weight basis to Keyhole Limpet Haemocyanin (KLH; Sigma, Poole, England) using the random glutaraldehyde method described above. This preparation was emulsified into Freunds Complete Adjuvant and administered sub-cutaneously into sheep (South Down x Kent), 1ml being divided between two sites on the flank of each sheep. The total initial dose was approximately 500μg peptide per sheep. There were 5 sheep per treatment including a negative control group of KLH only. Further similar immunisations were made at 28 and 70 days post initial dosage. These contained about 50 and 5μg peptide per sheep respectively.

All animals were blood sampled into serum 'Monovette' tubes, by jugular venepuncture on days -5, +14, +27, +35, +42, +56, +63, +70, +84, +98 and +112 days (relative to the first immunisation). In addition larger samples of up to 200 mls were taken at appropriate occasions for subsequent preparation of antibodies. The sera from all samples were separated and stored at -20°Cprior to being tested for the presence of anti-(human)GRF antibodies by competitive radioimmunassay as described above.

Results

All sheep survived the immunisations. The table describes the number of animals which produced a positive antibody response in at least two of the test bleeds:

| Peptide | Positive Responders % |
|---|---|
| I | 40 |
| II | 20 |
| III | 40 |
| IV | 60 |
| V | 40 |
| VI | 20 |
| VII | 60 |
| VIII | 0 |
| negative control | 0 |

Peptide VIII is a considerable departure from the human GRF sequence and more closely resembles that of ovine/bovine/porcine GRF sequences. Dot-blot analysis (Harlow and Lane, 1988) revealed that antibodies to this antigen had been raised in 60% of the animals and would be likely to recognise ovine and other GRF's.

Conclusions

Antibodies can be raised to a range of peptides from the GRF sequence and these will recognise the intact molecule from which they were derived and/or the original immunogen.

2. In vitro GRF-potentiating activity of antisera

Method

Using an in vitro system based upon that described by Machlin et al (1974) but using pituitaries from sheep, the action of GRF with or without different types of ovine antisera was investigated. GH in the test medium was assayed by the method of Hart et al (1975) except that the standards were dissolved in the appropriate in vitro medium and anti-guinea-pig "Sac-cel" (Wellcome Diagnostics) was used instead of the carrier/double antibody system described.

Antisera (5µl) containing hpGRF at 1000µg/ml and less as appropriate (or not) was added to quadruplicate wells containing confluent pituitary cells and 1ml of fresh medium in which the foetal calf serum had been omitted. The medium was removed after 4 hours at 37°C and frozen at -20°C for subsequent GH assay.

Results

The table shows the amounts of GH detected in the in vitro media by radioimmuno assay. They are expressed as µgGH/ml/5 hours medium with standard deviations.

| | GRF 1-44 added (µg/ml) in anti-serum | | | | |
|---|---|---|---|---|---|
| Sheep Anti-Sera to | 0 | 1.0 | 10.0 | 100.0 | 1000.0 |
| hpGRF 1-29 glutaraldehyde conjugation to ovalbumin | <1.0 | <1.0 | <1.0 | 14.9 ± 11.0 | 25.6 ± 9.4 |
| Carrier only glutaraldehyde cross-linked ovalbumin | <1.0 | 30.2 ± 7.9 | 61.7 ± 8.6 | 82.9 ± 16. | 103.4 ± 11.1 |
| hpGRF Cys + 30-44-NH$_2$ MBS conjugation to ovalbumin | <1.0 | 63.1 ± 9.1 | 79.2 ± 10.9 | 101.3 ± 10.1 | 99.7 ± 14.3 |

Conclusions

These results show that antibodies to the region 30-44 of the GRF molecule can enhance its activity in vitro, whereas those raised to the region 1-29 will diminish the activity of added GRF. The enhancement indicated from these data is approximately seven- to ten-fold.

## 3. Purification of Antibodies from Antisera

The serum from the large volume bleeding sessions using selected sheep was purified to yield a primarily antibody fraction which could be used in the subsequent in vivo experiment. This was based upon that described by Harlow and Lane (1988): A two stage ammonium sulphate fractionation yielded a semi-pure fraction which was dialysed against 5mM sodium phosphate (pH6.5), prior to batch purification using a DEAE matrix. The final preparation was re-assayed for activity and stored, frozen at -20°C, in PBS without preservatives.

## 4. In vivo GRF-potentiating activity of anti-GRF antibodies

### Method

Fifteen sheep (maiden Scottish half-bred ewes) with a mean liveweight of 48kg were prepared with unilateral in-dwelling carotid and jugular catheters, 24 hours prior to the first experimental period. They had been adjusted for two weeks to a 16% crude protein, complete, pelleted diet fed at a rate of 3.5% of liveweight. Food was withdrawn 18 hours prior to, and during the sampling phases of the experiment.

The basic design was a Latin square for treatment order for each sheep, with three basic treatments. These treatments were:
(1) GRF [human 1-44 amide, Cambridge Research Biochemicals] alone at 1μg/kg;
(2) Antibodies only [equivalent to approximately a ten-fold excess over the GRF dose];
(3) GRF at 1μg/kg previously complexed with antibodies [at a ten-fold excess as above] by mixing gently for one hour at room temperature.

There were antibodies from three different antisera directed to GRF sequences 1-14, 31-44 or 35-44 which were randomly allocated to the sheep on test, at 5 animals per group.

Blood samples were taken from the venous catheter at ten minute intervals prior to administration (and flushing in) of the GRF and/or antisera preparations via the carotid catheter at 'time 0'. Six further venous samples were taken at five minute intervals (to t + 30 min); at 10 minute intervals until t + 60 min and finally eight samples at 30 minute intervals (to t + 240min). Before retaining 5ml venous blood into heparinised tubes, the initial 2ml was discarded. Samples were centrifuged and the plasma separated and stored at -20°C until assayed for growth hormone levels by radioimmunassay (Hart et al., 1975). Subsequent treatments of the design were completed at 7 and 14 days later.

Statistical: The mean of individual GH levels were calculated for each time point and analyzed by analysis of variance and an F-protected t-test. The area under each curve (or parts thereof) was calculated using Simpson's rule for integration and these were compared similarly (SAS, 1985).

### Results

The meaned responses for each treatment group for the administration of each of the antibody preparations raised to GRF 1-14, 31-44 and 35-44 are shown in Figures One to Three respectively. Statistical parameters have been omitted for clarity.

It can be seen that the GRF gives a GH response curve within a few minutes after administration which returns to near control levels within the test period. The GRF 1-14 antibodies have abolished this response in the complexed preparation and do not appear to be associated with a major episode of GH release when given alone. In remarkable contrast, the complex of GRF and antibodies to sequences 31-44 or 35-44 give a plasma GH level consistently elevated above that from GRF alone. Additionally, the duration of these elevated GH levels may also be more sustained, as in Figure Three, around 90-210 minutes.

The development of an anti-GRF response is shown by the titration curves for an immunised animal in Figure Four.

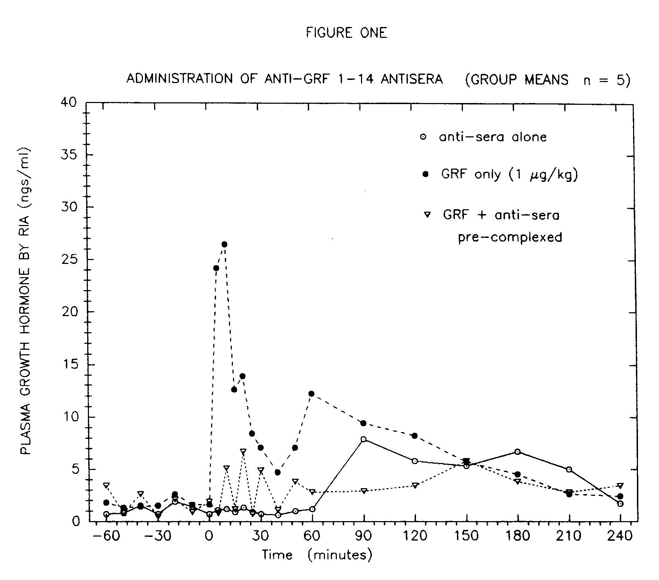

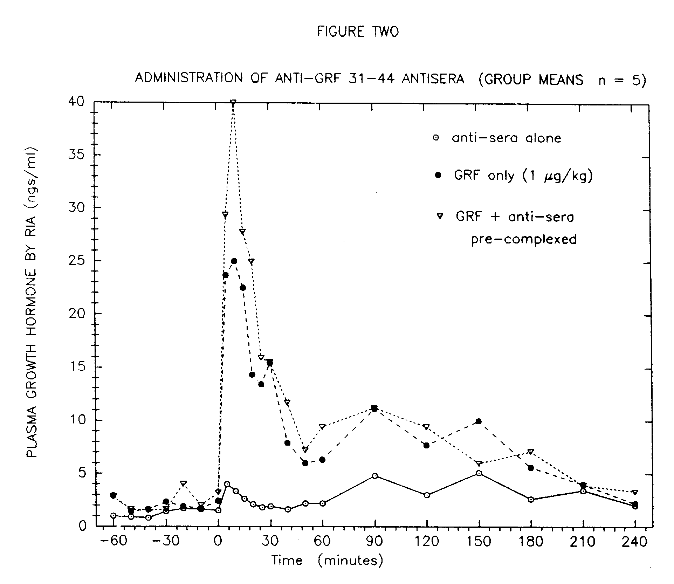

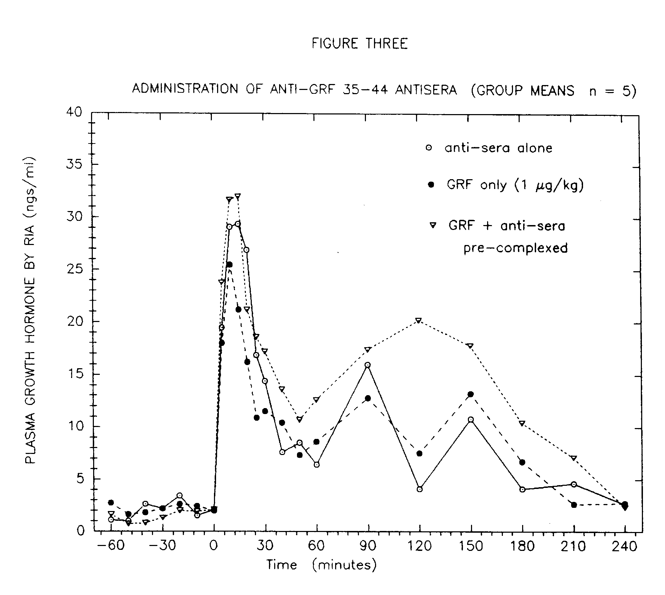

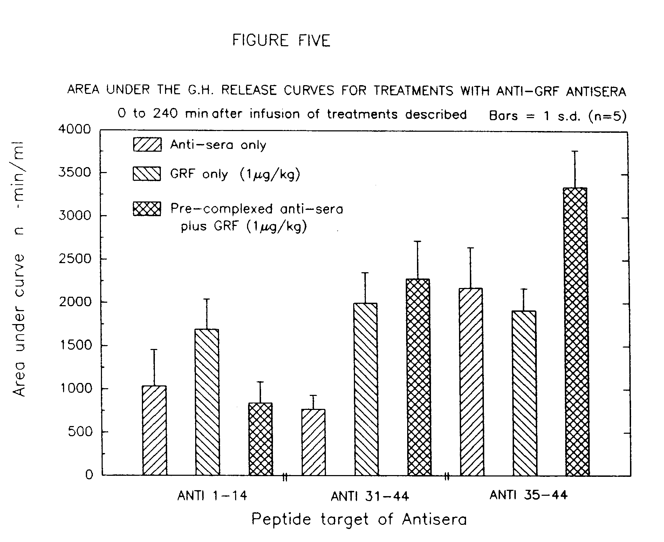

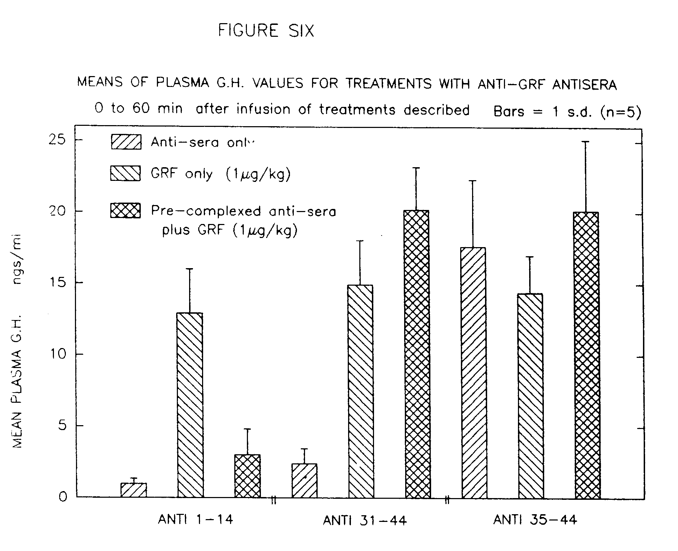

Figure 5 summarises these patterns of activity as means of areas under the curves in Figures 1-3 derived for each animal on test. This Figure also highlights the capacity of the antibodies to GRF 35-44 to inherently initiate a pulse of GH. Figure 6 emphasizes the activity during the first 60 minutes post treatment, demonstrating a significant difference (p <0.01) of mean plasma GH levels between the 1-14 and 31-44 anti-sera, as well as the similar enhancement effects of the GRF complexed with antibodies to 31-44 and 35-44 which are also highly statistically significant (p <0.01).

Overall Conclusions

Antibodies to the region 1-14 of the GRF sequence will inhibit the effects of GRF, but in suprising contrast those raised to regions of the GRF molecule between residues 31 and 44 not only do not inhibit but are able to enhance the biological activity of GRF. These antibodies can be used to increase the Growth Hormone releasing capabilities of GRF either from endogenous sources or exogenous administration, including use in sustained release devices et cetera or in transgenic animals expressing additional GRF or GH genes.

## Appendix I

### Amino acid symbols

| Amino acid | Three-letter symbol | One-letter symbol |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

References

Armstrong, J.D., Esbenshade, K.L., Coffrey, M.T., Heimer, E., Campbell, R., Mowles, T. and Felix, A. (1989). Biotechnology in Growth Regulation. Editors, R.B. Heap, C.G. Prosser and G.E. Lamming. Published by Butterworth & Co. Ltd.

Aston, R., Cooper, L., Holder, A.T., Ivanyi, J. and Preece, M.A. (1985) Molec. Immunol. 22: 271-275

Chard, T.(1987). An Introduction to Radioimmunoassay and Related Techniques. 3rd Edition. Elsevier, Amsterdam.

Enright, W.J., Chapin, L.T., Moseley, W.M., Zinn, S.A., and Tucker, H. Allen (1986).
Journal of Dairy Science 69: 344-351.

Felix, A.M., Heimer, E.P., and Mowles, T.F., (1985).
Annual Reports in Medicinal Chemistry 20: 185-192.

Guillemin, R., Brazeau, P., Bohlen, P., Esch, F., Ling, N., and Wehrenberg, W.B. (1982).
Science 218: 585- 587.

Harlow, E. and Lane, D. (1988). Antibodies: A Laboratory Manual. Published by Cold Spring Harbour Laboratory, New York.

Hart, I.C., Flux, D.S., Andrews, P., and A.S. McNeilly (1975). Hormone and Metabolic Research 7 (1): 35-40.

Hart, I.C., James, S., Perry, B.N., and Simmonds, A.D. (1984). Journal of Endocrinology 103: 173-178.

Lance, V.A., Murphy, W.A., Sueiras-Diaz, J., and Coy, D.H. (1984). Biochemical and Biophysical Research Communication 119 (1): 265-272.

Lerner, R.A., Green, N., Alexander, H., Liu, F-T., Sutcliffe, J.G., and Shinnick (1981), T.M. Proc. Natl. Acad. Sci. USA, 78: 3403-3407.

Machlin, L.J., Jacobs, L.S., Cirulis, N., Kimes, R., and Miller, R. (1974). Endocrinology 95: 1350-1357.

Marglin and Merrifield (1970). Ann. Rev. Biochem. 39 841-866 at 862.

Moseley, W.M., Huisman, J., and Weerden, E.J. (1987).
Domestic Animal Endocrinology 4 (1): 51-59.

Reynolds, C.M.M., Buttery, P.J., Haynes, N.B., and Huskisson, N. (1989). Biotechnology in Growth Regulation. Editors, R.B. Heap, C.G. Prosser and G.E. Lamming. Published by Butterworth & Co. Ltd.

Rivier, J Spiess, J., Thorner, M., and Vale, W. (1982).
Nature, London 300: 276-278.

SAS (1985) SAS User's Guide: Statistics, Version 5. Published by SAS Institute Inc., Cary, N.C., U.S.A.

Stewart et al (1969). In: Solid Phase Peptide Synthesis. Published by W.H. Freeman, San Francisco.

**Claims**

**Claims for the following Contracting States : AT, BE, CH, LI, DE, DK, FR, GB, IT, LU, NL, SE**

1. A pharmaceutical antigenic formulation comprising a peptide fragment of vertebrate growth hormone releasing factor (GRF) from the region spanning positions 28-44 thereof, or a salt, ester, amide or protected form of said peptide and means to provide adjuvant and carrier functions, wherein said peptide is capable of raising antibodies in a vertebrate to increase or enhance the activity of GRF in said vertebrate.

2. A formulation according to claim 1 wherein the peptide corresponds to the fragment of GRF
   (a) GRF 31-44 or
   (b) GRF 35-44
   wherein the GRF is of human, bovine, ovine, porcine, caprine, equine, canine, feline, avian or salmon origin, or a salt thereof.

3. A formulation according to claim 1 wherein the peptide additionally comprises an N-terminal cysteine residue.

4. A formulation according to claim 1 wherein the peptide is conjugated to an antigen.

5. A formulation according to claim 1 wherein the peptide is conjugated to (a) itself, (b) another peptide as defined in any one of claims 1 to 3, (c) a T-cell epitope or (d) part or all of the somatostatin or growth hormone molecules.

6. A formulation according to claim 1 wherein the peptide is linked to a carrier.

7. A formulation according to claim 1 wherein the peptide is mixed with an adjuvant.

EP 0 405 763 B1

8. A formulation according to claim 1 wherein the peptide is fused into a protein encoded by a micro-organism.

9. A vertebrate growth hormone releasing factor (GRF) complexed with anti-GRF antibodies specifically directed to a peptide as defined in any of claims 1 to 3.

10. A non-therapeutic method of enhancing somatogenesis, lactogenesis or carcass-composition of a non-human vertebrate comprising administering to the vertebrate a formulation according to any one of claims 1 to 8 or a complex according to claim 9.

11. The peptides defined in claim 2 and claim 3.

12. A process for the preparation of a peptide according to claim 11 by chemical peptide synthesis or by cultivating a suitably transformed organism.

13. A polynucleotide sequence encoding a peptide according to claim 11.

**Claims for the following Contracting States : ES, GR**

1. A process for the preparation of a pharmaceutical antigenic formulation comprising bringing into association a peptide fragment of vertebrate growth hormone releasing factor (GRF) from the region spanning positions 28-44 thereof, or a salt, ester, amide or protected form of said peptide and means to provide adjuvant and carrier functions, wherein said peptide is capable of raising antibodies in a vertebrate to increase or enhance the activity of GRF in said vertebrate.

2. A process according to claim 1 wherein the peptide corresponds to the fragment of GRF
    (a) GRF 31-44 or
    (b) GRF 35-44
wherein the GRF is of human, bovine, ovine, porcine, caprine, equine, canine, feline, avian or salmon origin, or a salt thereof.

3. A process according to claim 1 wherein the peptide additionally comprises an N-terminal cysteine residue.

4. A process according to claim 1 wherein the peptide is conjugated to an antigen.

5. A process according to claim 1 wherein the peptide is conjugated to (a) itself, (b) another peptide as defined in any one of claims 1 to 3, (c) a T-cell epitope or (d) part or all of the somatostatin or growth hormone molecules.

6. A process according to claim 1 comprising the step of linking the peptide to a carrier.

7. A process according to claim 1 comprising the step of mixing the peptide with an adjuvant.

8. A process according to claim 1 wherein the peptide is fused into a protein encoded by a micro-organism.

9. A process for the preparation of a hormone-antibody complex characterised in that vertebrate growth hormone releasing factor (GRF) is complexed with anti-GRF antibodies specifically directed to a peptide as defined in any of claims 1 to 3.

10. A non-therapeutic method of enhancing somatogenesis, lactogenesis or carcass-composition of a non-human vertebrate comprising administering to the vertebrate a formulation prepared according to any one of claims 1 to 8 or a complex prepared according to claim 9.

11. A process for the preparation of the peptides defined in claim 2 and claim 3 by chemical peptide synthesis or by cultivating a suitably transformed organism.

13

**Patentansprüche**
**Patentansprüche für folgende Vertragsstaaten : AT, BE, CH, LI, DE, DK, FR, GB, IT, LU, NL, SE**

1. Pharmazeutische antigene Formulierung, umfassend ein Peptidfragment eines Wirbeltier-Wachstumshormon-Releasing-Faktors (GRF) aus der Region, umfassend die Positionen 28-44 davon, oder ein Salz, ein Ester, ein Amid oder eine geschützte Form des Peptids und Mittel zur Bereitstellung von Adjuvans- und Trägerfunktionen, wobei das Peptid Antikörper in einem Wirbeltier erzeugen kann, um die Aktivität des GRFs in dem Wirbeltier zu erhöhen oder zu verstärken.

2. Formulierung nach Anspruch 1, worin das Peptid dem GRF-Fragment von
    (a) GRF 31-44 oder
    (b) GRF 35-44
    oder einem Salz davon entspricht, wobei der GRF von Menschen, Rindern, Schafen, Schweinen, Ziegen, Pferden, Hunden, Katzen, Vögeln oder Lachsen stammt.

3. Formulierung nach Anspruch 1, worin das Peptid zusätzlich einen N-terminalen Cysteinrest umfaßt.

4. Formulierung nach Anspruch 1, worin das Peptid an ein Antigen konjugiert ist.

5. Formulierung nach Anspruch 1, worin das Peptid an (a) sich selbst, (b) ein anderes Peptid nach einem der Ansprüche 1 bis 3, (c) ein T-Zell-Epitop oder (d) einen Teil oder die Gesamtheit der Somatostatin- oder Wachstumshormonmoleküle konjugiert ist.

6. Formulierung nach Anspruch 1, worin das Peptid an einen Träger gebunden ist.

7. Formulierung nach Anspruch 1, worin das Peptid mit einem Adjuvans vermischt ist.

8. Formulierung nach Anspruch 1, worin das Peptid in ein Protein, das von einem Mikroorganismus codiert wird, fusioniert ist.

9. Wirbeltier-Wachstumshormon-Releasing-Faktor (GRF), komplexiert mit Anti-GRF-Antikörpern, die spezifisch gegen ein Peptid nach einem der Ansprüche 1 bis 3 gerichtet sind.

10. Nicht-therapeutisches Verfahren zur Verstärkung der Somatogenese, Lactogenese oder Schlachtkörperzusammensetzung eines nicht-menschlichen Wirbeltieres, wobei dem Wirbeltier eine Formulierung nach einem der Ansprüche 1 bis 8 oder ein Komplex nach Anspruch 9 verabreicht wird.

11. Peptide nach Anspruch 2 und 3.

12. Verfahren zur Herstellung eines Peptids nach Anspruch 11 durch chemische Peptidsynthese oder durch Züchtung eines geeigneterweise transformierten Organismus.

13. Polynucleotidsequenz, codierend ein Peptid nach Anspruch 11.

**Patentansprüche für folgende Vertragstaaten : ES, GR**

1. Verfahren zur Herstellung einer pharmazeutischen antigenen Formulierung, wobei man ein Peptidfragment eines Wirbeltier-Wachstumshormon-Releasing-Faktors (GRF) aus der Region, umfassend die Positionen 28-44 davon, oder ein Salz, einen Ester, ein Amid oder eine geschützte Form des Peptids und Mittel zur Bereitstellung von Adjuvans- und Trägerfunktionen assoziiert, wobei das Peptid Antikörper in einem Wirbeltier erzeugen kann, um die Aktivität von GRF in dem Wirbeltier zu erhöhen oder zu verstärken.

2. Verfahren nach Anspruch 1, wobei das Peptid dem GRF-Fragment von
    (a) GRF 31-44 oder
    (b) GRF 35-44
    oder einem Salz davon entspricht, wobei der GRF von Menschen, Rindern, Schafen, Schweinen, Ziegen, Pferden, Hunden, Katzen, Vögeln oder Lachsen stammt.

**3.** Verfahren nach Anspruch 1, wobei das Peptid zusätzlich einen N-terminalen Cysteinrest umfaßt.

**4.** Verfahren nach Anspruch 1, wobei das Peptid an ein Antigen konjugiert wird.

**5.** Verfahren nach Anspruch 1, wobei das Peptid an (a) sich selbst, (b) ein anderes Peptid nach einem der Ansprüche 1 bis 3, (c) ein T-Zell-Epitop oder (d) einen Teil oder die Gesamtheit der Somatostatin- oder Wachstumshormonmoleküle konjugiert wird.

**6.** Verfahren nach Anspruch 1, umfassend die Stufe des Knüpfens des Peptids an einen Träger.

**7.** Verfahren nach Anspruch 1, umfassend die Stufe des Vermischens des Peptids mit einem Adjuvans.

**8.** Verfahren nach Anspruch 1, wobei das Peptid in ein Protein, das von einem Mikroorganismus codiert wird, fusioniert wird.

**9.** Verfahren zur Herstellung eines Hormon-Antikörper-Komplexes, **dadurch gekennzeichnet,** daß der Wirbeltier-Wachstumshormon-Releasing-Faktor (GRF) mit Anti-GRF-Antikörpern, die spezifisch gegen ein Peptid nach einem der Ansprüche 1 bis 3 gerichtet sind, komplexiert wird.

**10.** Nicht-therapeutisches Verfahren zur Verstärkung der Somatogenese, Lactogenese oder Schlachtkörperzusammensetzung eines nicht-menschlichen Wirbeltieres, wobei dem Wirbeltier eine Formulierung nach einem der Ansprüche 1 bis 8 oder ein Komplex nach Anspruch 9 verabreicht wird.

**11.** Verfahren zur Herstellung eines Peptids nach Anspruch 2 und Anspruch 3 durch chemische Peptidsynthese oder durch Züchtung eines geeigneterweise transformierten Organismus.

**Revendications**
**Revendications pour les Etats contractants suivants : AT, BE, CH, LI, DE, DK, FR, GB, IT, LU, NL, SE**

**1.** Formulation pharmaceutique antigénique comprenant un fragment peptidique du facteur ayant une activité libératrice de l'hormone de croissance (GRF) d'un vertébré issu de la région s'étendant des positions 28 à 44, ou un sel, ester, amide ou une forme protégée dudit peptide, et des moyens pour remplir les fonctions d'adjuvant et de véhicule, dans laquelle ledit peptide est capable d'élever le taux d'anticorps chez un vertébré pour augmenter ou améliorer l'activité du GRF chez ledit vertébré.

**2.** Formulation selon la revendication 1, dans laquelle ledit peptide correspond au fragment de GRF
    (a) GRF 31-44 ou
    (B) GRF 35-44
dans lequel le GRF est d'origine humaine, bovine, ovine, porcine, caprine, équine, canine, féline, aviaire ou de saumon, ou un de ses sels.

**3.** Formulation selon la revendication 1, dans laquelle le peptide comprend aussi un résidu de cystéine N-terminal .

**4.** Formulation selon la revendication 1, dans laquelle le peptide est conjugué à un antigène.

**5.** Formulation selon la revendication 1, dans laquelle le peptide est conjugué (a) à lui-même, (b) à un autre peptide tel que défini dans l'une quelconque des revendications 1 à 3, (c) à un épitope de cellules T ou (d) à une partie ou à la totalité des molécules de somatostatine ou d'hormone de croissance.

**6.** Formulation selon la revendication 1, dans laquelle le peptide est lié à un véhicule.

**7.** Formulation selon la revendication 1, dans laquelle le peptide est mélangé à un adjuvant.

**8.** Formulation selon la revendication 1, dans laquelle le peptide est fusionné en une protéine codée par un microorganisme.

15

9. Facteur ayant une activité libératrice de l'hormone de croissance (GRF) d'un vertébré complexé avec des anticorps anti-GRF spécifiquement dirigés contre un peptide tel que défini dans l'une quelconque des revendications 1 à 3.

10. Procédé non thérapeutique pour améliorer la somatogénèse, la lactogénèse ou la composition d'une carcasse d'un vertébré non humain, comprenant l'administration au vertébré d'une formulation selon l'une quelconque des revendications 1 à 8 ou d'un complexe selon la revendication 9.

11. Peptides définis dans la revendication 2 et la revendication 3.

12. Procédé de préparation d'un peptide selon la revendication 11 par synthèse peptidique chimique ou par culture d'un organisme transformé de façon adéquate.

13. Séquence polynucléotidique codant pour un peptide selon la revendication 11.

**Revendications pour les Etats contractants suivants : ES, GR**

1. Procédé de préparation d'une formulation pharmaceutique antigénique comprenant l'association d'un fragment peptidique du facteur ayant une activité libératrice de l'hormone de croissance (GRF) d'un vertébré issu de la région s'étendant des positions 28 à 44, ou d'un sel, ester, amide ou d'une forme protégée dudit peptide, et des moyens pour remplir les fonctions d'adjuvant et de véhicule, dans lequel ledit peptide est capable d'élever le taux d'anticorps chez un vertébré pour augmenter ou améliorer l'activité du GRF chez ledit vertébré.

2. Procédé selon la revendication 1, dans lequel ledit peptide correspond au fragment de GRF
    (a) GRF 31-44 ou
    (B) GRF 35-44
dans lequel le GRF est d'origine humaine, bovine, ovine, porcine, caprine, équine, canine, féline, aviaire ou de saumon, ou un de ses sels.

3. Procédé selon la revendication 1, dans lequel le peptide comprend aussi un résidu de cystéine N-terminale.

4. Procédé selon la revendication 1, dans lequel le peptide est conjugué à un antigène.

5. Procédé selon la revendication 1, dans lequel le peptide est conjugué (a) à lui-même, (b) à un autre peptide tel que défini dans l'une quelconque des revendications 1 à 3, (c) à un épitope de cellules T ou (d) à une partie ou à la totalité des molécules de somatostatine ou d'hormone de croissance.

6. Procédé selon la revendication 1, dans lequel le peptide est lié à un véhicule.

7. Procédé selon la revendication 1, dans lequel le peptide est mélangé à un adjuvant.

8. Procédé selon la revendication 1, dans lequel le peptide est fusionné en une protéine codée par un microorganisme.

9. Procédé de préparation d'un complexe hormone-anticorps, caractérisé en ce que le facteur ayant une activité libératrice de l'hormone de croissance (GRF) d'un vertébré est complexé avec des anticorps anti-GRF spécifiquement dirigés contre peptide tel que défini dans l'une quelconque des revendications 1 à 3.

10. Procédé non thérapeutique pour améliorer la somatogénèse, la lactogénèse ou la composition d'une carcasse d'un vertébré non humain, comprenant l'administration au vertébré d'une formulation préparée selon l'une quelconque des revendications 1 à 8 ou d'un complexe préparé selon la revendication 9.

11. Procédé de préparation des peptides définis dans la revendication 2 et la revendication 3, par synthèse peptidique chimique ou par culture d'un organisme transformé de façon adéquate.

FIGURE ONE

ADMINISTRATION OF ANTI-GRF 1-14 ANTISERA    (GROUP MEANS  n = 5)

FIGURE TWO

ADMINISTRATION OF ANTI—GRF 31—44 ANTISERA (GROUP MEANS n = 5)

FIGURE THREE

ADMINISTRATION OF ANTI−GRF 35−44 ANTISERA (GROUP MEANS n = 5)

## FIGURE FOUR

### Titration curves of sera from an immunised sheep

FIGURE FIVE

AREA UNDER THE G.H. RELEASE CURVES FOR TREATMENTS WITH ANTI–GRF ANTISERA

0 to 240 min after infusion of treatments described    Bars = 1 s.d. (n=5)

21

## FIGURE SIX

MEANS OF PLASMA G.H. VALUES FOR TREATMENTS WITH ANTI—GRF ANTISERA

0 to 60 min after infusion of treatments described    Bars = 1 s.d. (n=5)